# EUROPEAN PATENT SPECIFICATION

(11) **EP 2 478 909 B1**
(45) Date of publication and mention of the grant of the patent: **25.04.2018**
(21) Application number: 10817343.6
(22) Date of filing: 17.06.2010
(51) Int. Cl.: A61K 9/20, A61K 31/59, A61K 31/675, A61P 19/10, A61K 9/16

(54) **PHARMACEUTICAL COMPOSITIONS COMPRISING BISPHOSPHONATE DERIVATIVES AND HIGH-DOSE CHOLECALCIFEROL**
PHARMAZEUTISCHE ZUSAMMENSETZUNGEN MIT BISPHOSPHONATDERIVATEN UND HOCH DOSIERTEM CHOLECALCIFEROL
COMPOSITIONS PHARMACEUTIQUES COMPRENANT DES DÉRIVÉS DE BISPHOSPHONATE ET DU CHOLÉCALCIFÉROL À DOSE ÉLEVÉE

(30) Priority: 18.09.2009 KR 20090088480
(43) Date of publication of application: 25.07.2012
(73) Proprietor: Hanlim Pharmaceutical Co., Ltd., Gyeonggi-do 449-935 (KR)
(72) Inventor: KIM, Jin-Sun, Yongin-si Gyeonggi-do 446-720 (KR); LEE, Geun-Hyeog, Yongin-si Gyeonggi-do 446-722 (KR); CHOI, Byong-Sun, Seoul 150-094 (KR); KIM, Jae-Shin, Seoul 138-797 (KR); PARK, Jin-Ha, Anyang-si Gyeonggi-do 431-070 (KR); O, Mi-Jin, Hwaseong-si Gyeonggi-do 445-777 (KR); KIM, Eun-Mi, Seoul 152-052 (KR)
(74) Representative: Turner, Craig Robert
(86) International application number: PCT/KR2010/003920
(87) International publication number: WO 2011/034274

(56) References cited:
- EP-A2- 2 581 086
- WO-A1-2005/117906
- WO-A1-2008/026907
- JP-B2- 2 963 718
- KR-B1- 100 844 256
- US-A- 5 328 903
- US-A1- 2003 195 171
- US-A1- 2005 176 685
- US-A1- 2005 261 250
- JEAN, G. ET AL.: 'Monthly cholecalciferol administration in haemodialysis patients: a simple and efficient strategy for vitamin D supplementation.' NEPHROL. DIAL. TRANSPLANT vol. 24, 21 July 2009, pages 3799 - 3805, XP008153118

## Description

### TECHNICAL FIELD

The prevent invention relates to a pharmaceutical composition for preventing or treating osteoporosis to be administered once a month, the pharmaceutical composition comprising a bisphosphonate derivative, such as risedronic acid (or its salt) or ibandronic acid (or its salt), and cholecalciferol in a high-dose, i.e. in an amount corresponding to 24,000 to 50,000 IU.

### BACKGROUND ART

Osteoporosis, one of the metabolic diseases, causes bone mass reduction and bone micro-architectural alterations, due to which bone damage is likely to occur, thereby ultimately leading to risks of fracture. Osteoporosis is prevalent in elderly people and an increase in complications associated with fracture and treatment costs therefor lead to a high social burden. To treat osteoporosis, bisphosphonate-based medicines inhibiting bone resorption have been clinically used. Examples of such medicines are pamidronic acid, alendronic acid, risedronic acid, ibandronic acid, zoledronic acid, and salts thereof.

However, it has been reported that the bisphosphonate-based medicines are associated with hypocalcemia. Bisphosphonates inhibit bone resorption of osteoclasts, thereby inhibiting leakage of calcium from the bone, and thus, calcium concentration in the blood is reduced. The reduction of calcium concentration in the blood homeostatically increases parathyroid hormone level in the blood. Since hypocalcemia is very dangerous to a patient that is receiving a therapy for vitamin D deficiency, parathyroid dysfunction, renal malfunction, or Paget's disease, an appropriate intake of calcium and vitamin D is necessary before and during the bisphosphonate therapy (Recker RR, Lewiecki EM, Miller PD, Reiffel J. Safety of bisphosphonates in the treatment of osteoporosis: Am J Med. 2009 Feb; 122(2Supp):S22-32).

In response to the increase of calcium requirement according to the calcium influx into bone which results from the bisphosphonate therapy, intestinal absorption of calcium should be raised for accomplishing effective treatment of osteoporosis. In this regard, a vitamin D level in the blood is an important factor. Accordingly, in osteoporosis therapies by administering bisphosphonate-based medicine, intake of vitamin D or a derivative thereof is recommended. Typically, vitamin D is administered in the amount of 400IU to 800IU per day. For example, KR 2005-0110814 discloses a pharmaceutical composition, comprising: a bisphosphonate, pharmaceutically acceptable salts, derivatives or hydrates of the bisphosphonate, or mixtures thereof; and a vitamin D compound.

Vitamin D increases calcium absorption in the small intestine and also plays an important role in differentiation of bone cells and generation of high quality bone matrix in osteoblasts. Accordingly, it is important to maintain the concentration of vitamin D metabolites in the blood at an appropriate level, for improving both bone metabolism and therapeutic effects against osteoporosis. Vitamin D is a fat-soluble vitamin, and a vitamin D deficiency may occur due to insufficient exposure to light or inappropriate dietary intake (Martindale: The complete drug reference, Thirty-fifth edition). The major biological function of vitamin D is to promote in-taken calcium absorption in the small intestine so as to control the blood calcium level within a normal range. Vitamin D and derivatives thereof (for example, cholecalciferol, ergocalciferol, calcitriol, doxercalciferol, maxacalcitol, paricalcitol, or the like) are used for treating rickettsial infections, osteomalacia, and hypocalcemia.

In 1997, the National Academy of Science (NAS) recommended a dietary intake of vitamin D: 200 IU for 19-50 aged adults, 400 IU for 51-70 aged adults, and 600 IU for 71 or more aged adults. In 2007, the Korean Society of Bone Metabolism recommended 800IU as a dietary intake of vitamin D. The recommended dietary intake of vitamin D is determined in consideration of factors that affect the vitamin D level in the blood, such as exposure to sunlight and body fat. 25-Hydroxyvitamin D, a circulatory metabolite of vitamin D, is known as the most stable marker of vitamin D (Whiting SJ, Calvo MS. Dietary recommendations for vitamin D: J Nutr 2005; 135: 304-9). For maintaining good health, it is known that a minimum concentration of vitamin D in the blood is 30 ng/mL as a concentration corresponding to 25-hydroxyvitamin D and that an optimal concentration thereof is in the range of 36 to 40 ng/mL as a concentration corresponding to 25-hydroxyvitamin D (Am J Clin Nutr 2006, 84:18-28, Estimation of optimal serum concentration of 25-hydroxyvitamin D for multiple health outcomes).

However, an excess intake of vitamin D or derivatives thereof may cause hypercalcemia syndrome or calcium poisoning. Occasionally occurring acute side effects include appetite loss, headache, vomiting, constipation, or the like, and chronic symptoms include malnutrition, paraesthesia, fever accompanied with thirst, hyperuresis, dehydration, indifference, growth delay, urinary tract infection, or the like. And also, it is known that the therapeutic dose of vitamin D or derivatives thereof is very slightly different from the dose causing hypercalcemia, and that the dosage thereof needs to be adjusted very carefully. In case of adults, a daily intake of 1.25-2.5 mg (50,000IU-100,000IU of vitamin D) of ergocalciferol causes hypervitaminosis D (a state of vitamin D toxicity), and this risk is very high due to long half-life and metabolic pathway of tissue accumulation of ergocalciferol (AHFS Drug information, 2007).

KR 10-0822133 has disclosed a complex formulation comprising a bisphosphonate and vitamin D or a derivative thereof, wherein vitamin D or its derivative is formulated into a solid dispersion so as to improve the stability thereof. That is, this patent discloses a complex formulation for preventing or treating osteoporosis, which comprises (i) a solid dispersion comprising vitamin D or its derivative and a cyclodextrin, and (ii) a bisphosphonate.

KR10-0844256 has disclosed a pharmaceutical composition for treating metabolic bone diseases comprising risedronate and a vitamin D compound. The composition comprises a tablet comprising risedronate coated with a delayed release layer comprising an enteric polymer, and coated with coating comprising the vitamin D compound and a water-soluble polymer.

Also, KR 10-0317935 has disclosed a pharmaceutical composition comprising 1,000 to 50,000 parts by weight of alendronate per one part by weight of calcitriol. According to KR 10-0317935, the miscibility test between calcitriol and alendronate showed that the mixture of the two components resulted in deterioration of the stability of calcitriol. Therefore, in order to avoid the direct contact between calcitriol and alendronate, calcitriol and alendronate are independently diluted with mannitol to produce the respective granules, which are mixed and then formulated into, for example, a tablet or the like. And also, in order to maintain uniformity in the amount of ingredients when preparing a tablet, 50 to 60% (preferably 52%) of the total mannitol was used for calcitriol granules and 40 to 50% (preferably 48%) of the total mannitol was used for alendronate granules, so that the two kinds of granules were adjusted to have equal amounts before mixing them together.

Recently, KR 10-2007-0038115 has disclosed a method for treating a bone disorder, such as osteoporosis, which comprises administering an excessive amount of risedronate once a month. That is, this patent disclosed a method for treating or preventing a bone disorder in a human or other mammal in need thereof comprising orally administering to said human or other mammal a pharmaceutical composition comprising from about 65% to about 110% of the cumulative effective dose of risedronate according to a continuous dosing schedule of one, two, or three consecutive days per month.

Accordingly, it would be very desirable in terms of patients' drug compliance to develop a complex formulation to be administered once a month, the formulation comprising vitamin D or its derivative as well as a bisphosphonate-based medicine. However, as described above, the recommended dietary intake for vitamin D that is capable of avoiding side effects is as low as only 800 IU. Therefore, in case of designing a formulation to be administered once a month, there is a problem that the amount of vitamin D cannot be increased for obtaining a desired vitamin D level in the blood. And also, while the half life of vitamin D in the body is about two months, the half-life of calcitriol known as an active derivative of vitamin D is only 15 hours, which makes it very difficult to formulate calcitriol into a formulation to be administered once a month.

### DISCLOSURE

### Technical Problem

The present inventors performed various studies to develop a complex formulation to be administered once a month, which comprising a bisphosphonate-based medicine and vitamin D or its derivative thereof. From the fact that cholecalciferol shows a long half life (about 2 months) in the body unlike other vitamin D derivatives such as calcitriol, we designed a complex formulation of cholecalciferol and a bisphosphonate-based medicine, in particular, risedronic acid (or its salt) or ibandronic acid (or its salt). As a result, it was surprisingly found that, when a complex formulation obtained by using cholecalciferol in a much greater amount than the recommended daily dietary intake of 400 to 800 IU together with risedronic acid (or its salt) or ibandronic acid (or its salt) was administered once a month, bone resorption could be strongly suppressed, and at the same time, the concentration of 25(OH)D₃ in the blood could be maintained in an appropriate range, without showing any side effects such as hypercalcemia.

In addition, it was found that, when formulated into a complex formulation comprising an excessive amount of cholecalciferol through the use of a granule obtained by adsorbing cholecalciferol on microcrystalline cellulose as a carrier; and a bisphosphonate-based medicine, instability of cholecalciferol over time could be resolved, and at the same time, the amount of excipient could be substantially reduced, thereby resolving the problems caused by the large size of the resulting formulation (for example, tablet).

Accordingly, the present invention provides a pharmaceutical composition for preventing or treating osteoporosis to be administered once a month, the pharmaceutical composition comprising risedronic acid or its salt or ibandronic acid or its salt; and cholecalciferol in an excessive amount (i.e., in an amount corresponding to 24,000 to 50,000 IU) in a unit dosage form.

The present invention also provides a stabilized pharmaceutical composition for preventing or treating osteoporosis to be administered once a month, the pharmaceutical composition comprising 24,000 to 50,000 IU of cholecalciferol; and risedronic acid (or its salt) or ibandronic acid (or its salt).

### Technical Solution

In accordance with the present invention, there is provided a pharmaceutical composition for preventing or treating osteoporosis to be administered once a month, the pharmaceutical composition comprising: (a) cholecalciferol-containing granules obtained by adsorbing on microcrystalline cellulose a solution prepared by dissolving (i) cholecalciferol in an amount corresponding to 24,000 to 50,000 IU, preferably 30,000 IU; (ii) one or more selected from tocopheryl acetate, butylhydroxytoluene, and butylhydroxyanisol as a first stabilizing agent; and (iii) a binder in ethanol or an aqueous solution of ethanol, (b) mannitol as a second stabilizing agent, and (c) risedronic acid or its salt or ibandronic acid or its salt, wherein the components (a), (b) and (c) are homogenously mixed in the composition. The cholecalciferol-containing granules may be prepared by performing a granulation process comprising: mixing a solution prepared by dissolving cholecalciferol, the first stabilizing agent, and the binder in ethanol or the aqueous solution of ethanol with microcrystalline cellulose in a mixer to prepare a slurry, and then drying and milling the slurry. The first stabilizing agent is preferably a mixture of tocopheryl acetate and butylhydroxytoluene; and a weight ratio of tocopheryl acetate and butylhydroxytoluene may be in a range of 1 : 0.2 to 1.0. The first stabilizing agent may be present in an amount of 0.4 to 2.0 parts by weight based on 1 part by weight of cholecalciferol. The binder may be hydroxypropyl cellulose.

In the pharmaceutical composition of the present invention, the second stabilizing agent, i.e., mannitol, may be present in an amount of 5 to 20 wt% based on the total weight of the pharmaceutical composition.

The pharmaceutical composition of the present invention may further comprise one or more disintegrating agent selected from the group consisting of crospovidone, sodium starch glycolate, sodium carboxymethyl cellulose, and alginic acid; and one or more lubricant selected from the group consisting of magnesium stearate, stearic acid, talc, and silicon dioxide.

A dosage form of the pharmaceutical composition may be a tablet form, on which may comprise a film coating layer comprising a mixture of hydroxypropyl methylcellulose and polyethylene glycol, preferably having a weight ratio of hydroxypropyl methylcellulose and polyethylene glycol in a range of 5 to 15 : 1.

In accordance with an embodiment, there is provided a pharmaceutical composition in a tablet form consisting of (a) cholecalciferol-containing granules comprising 0.75 mg of cholecalciferol powder (40,000 IU/mg), 0.5 mg of butylhydroxytoluene, 0.9 mg of tocopheryl acetate, 6 mg of hydroxypropyl cellulose, and 40 mg of microcrystalline cellulose, (b) 40.0 mg of mannitol, (c) 150 mg of sodium risedronate or 150 mg of sodium ibandronate, and (d) 4 mg of crospovidone and 4 mg of magnesium stearate.

In accordance with another embodiment, there is provided a pharmaceutical composition in a film-coated tablet form comprising a film coating layer consisting of 5.1 mg of hydroxypropyl methylcellulose, 0.51 mg of polyethylene glycol 6000 (PEG 6000), 1.28 mg of titanium oxide, and 0.11 mg of talc on the tablet.

### ADVANTAGEOUS EFFECTS

The pharmaceutical composition of the present invention is a complex formulation of cholecalciferol having a long half life (about 2 months) in the body unlike other vitamin D derivatives such as calcitriol; and a bisphosphonate-based medicine, in particular, risedronic acid (or its salt) or ibandronic acid (or its salt), the complex formulation being able to be administered once a month. In particular, it is newly found by the present invention that, when both risedronic acid (or its salt) or ibandronic acid (or its salt); and cholecalciferol in a much greater amount, i.e., in an amount of 24,000 to 50,000 IU, than the recommended daily dietary intake (400 to 800 IU) were used for oral administration, both a synergistic effect can be expected and patient compliance can be improved in the treatment of osteoporosis, through suppressing bone resorption strongly and maintaining the concentration of vitamin D in the blood in an appropriate range, without showing any side effects such as hypercalcemia according to excessive administration of cholecalciferol.

In addition, the pharmaceutical composition of the present invention is formulated through the use of a granule obtained by adsorbing cholecalciferol on microcrystalline cellulose as a carrier; and a bisphosphonate-based medicine, instability of cholecalciferol over time can be resolved, and at the same time, the amount of excipient can be substantially reduced, thereby resolving the problems caused by the large size of the resulting formulation (for example, tablet). Accordingly, the pharmaceutical composition according to the present invention is a stabilized pharmaceutical composition, which can be administered one a month for preventing or treating osteoporosis, thereby increasing patients' drug compliance.

### BEST MODE

The term "risedronic acid or its salt" used herein refers to 3-pyridyl-1-hydroxyethylidene-1,1-bisphosphonic acid or its salt, and the salt includes a sodium salt, a potassium salt, a calcium salt, a magnesium salt, an ammonium salt, or the like, and preferably a sodium salt. Also, the risedronic acid or its salt, for example, sodium risedronate, includes various known forms such as an anhydrate form, a monohydrate form, a hemipentahydrate form, and various polymorphic forms. The amount of risedronic acid or its salt in the pharmaceutical composition according to the present invention may be a therapeutically effective amount, for example, in a range of about 100 to 165 mg, preferably about 150 mg, per unit dosage form such as a tablet, but not limited thereto.

Also, the term "ibandronic acid or its salt" used herein refers to 1-hydroxy-3-(N-methyl-N-pentyl)aminopropyl-1,1-diphosphonic acid or its salt, and the salt includes a sodium salt. The amount of ibandronic acid or its salt in the pharmaceutical composition according to the present invention may be a therapeutically effective amount, for example, in a range of about 100 to 165 mg, preferably about 150 mg, per unit dosage form such as a tablet, but not limited thereto.

"Cholecalciferol" is produced by ultraviolet radiation of its precursor 7-dehydrocholesterol present in the skin, and metabolized in the liver to calciferol and then metabolized in the kidney to calcitriol showing activity. In the pharmaceutical composition according to the present invention, cholecalciferol is used in an amount much greater than a typical dose (for example, 400 to 800 IU per day) of vitamin D. That is, the amount of cholecalciferol may be in a range of about 24,000 to 50,000 IU, preferably about 30,000 IU, per unit dosage form such as a tablet. 40 IU of cholecalciferol has a mass of about 1 Accordingly, an amount of cholecalciferol in a unit of mg corresponding to '24,000 to 50,000 IU' may be easily calculated by one of ordinary skill in the art. For example, 0.75 mg of commercially available cholecalciferol powder (40,000 IU/mg) corresponds to about 30,000 IU of cholecalciferol.

The pharmaceutical composition of the present invention is a complex formulation of cholecalciferol having a long half life (about 2 months) in the body unlike other vitamin D derivatives such as calcitriol; and a bisphosphonate-based medicine, in particular, risedronic acid (or its salt) or ibandronic acid (or its salt), the complex formulation being able to be administered once a month. In particular, it is newly found by the present invention that, when both risedronic acid (or its salt) or ibandronic acid (or its salt); and cholecalciferol in a much greater amount, i.e., in an amount of 24,000 to 50,000 IU, than the recommended daily dietary intake (400 to 800 IU) were used for oral administration, both a synergistic effect can be expected and patient compliance can be improved in the treatment of osteoporosis, through suppressing bone resorption strongly and maintaining the concentration of vitamin D in the blood in an appropriate range, without showing any side effects such as hypercalcemia according to excessive administration of cholecalciferol.

Accordingly, the present invention provides a pharmaceutical composition for preventing or treating osteoporosis to be administered once a month, the pharmaceutical composition comprising risedronic acid or its salt or ibandronic acid or its salt; and cholecalciferol in an amount corresponding to 24,000 to 50,000 IU, preferably 30,000 IU, in a unit dosage form. The unit dosage form may be a solid formulation form for oral administration, such as a tablet or a capsule. The unit dosage form is preferably a tablet form.

Meanwhile, cholecalciferol is very unstable over time. As a method for resolving this problem, applicability of the known stabilization method, e.g., the method for stabilizing a complex formulation of calcitriol and alendronate disclosed in KR 10-0317935 may be taken into consideration. However, if the method disclosed in KR 10-0317935 is applied, the risedronic acid (or its salt) or the ibandronic acid (or its salt) and cholecalciferol are independently granulated by performing separate two granulation processes, which complicate the manufacturing process, thereby increasing production costs. And also, the granulation processes require using excessive amounts of excipients (for example, in case of the composition according to KR 10-0317935, the total amount of mannitol with respect to the active ingredient is about 94.5% or more). In particular, if granules are prepared through the granulation processes using mannitol in order to secure content-uniformity and stability and then a tablet is formed from the obtained granules, the size of the resulting tablet becomes too big, so that it makes it difficult for patients to intake the tablet.

As a method for avoiding the problem described above, a commercially available cholecalciferol granule (100 IU/mg, butylhydroxytoluene is included, particle size: 150 or more (100 mesh), BASF company, German) may be taken into consideration. However, to incorporate cholecalciferol in an amount corresponding to 24,000 to 50,000 IU per unit dosage form, about 300 mg (for example, 30,000 IU of cholecalciferol) of cholecalciferol should be added to one tablet. Accordingly, when considering the amount of another active ingredient [about 150 mg/tablet of risedronic acid (or its salt) or ibandronic acid (or its salt)], the amounts of only the active ingredients is up to 450 mg/tablet. Besides, when an amount of an excipient (for example, mannitol), which is added to secure stability, is added thereto, the resulting tablet becomes at least 1,000 mg/tablet. Thus, the resulting tablet is too big, so that it makes it difficult for patients to intake the tablet. And also, the particle size of the cholecalciferol granule is as big as 150 or more (100 mesh). Therefore, when the cholecalciferol granule is formulated along with risedronic acid (or its salt) or ibandronic acid (or salt), it is difficult to secure content-uniformity. Further, poor compressibility (i.e., very low hardness) results in high friability and easy crumbling, during the tablet formation.

Accordingly, the present inventors performed various studies to develop a formulation method which is able to minimize the amount of an excipient, while maintaining the stability of cholecalciferol. As a result, it was surprisingly found that the use of a granule obtained by adsorbing cholecalciferol on microcrystalline cellulose as a carrier can resolve instability of cholecalciferol over time and at the same time, the amount of excipient could be substantially reduced (in particular, the amount of mannitol could be reduced to 20% or less), thereby resolving the problems caused by the large size of the resulting formulation (for example, tablet). And also, it was found that, since the obtained granule has excellent flowability, the content-uniformity could be maintained appropriately during the formulation process along with mannitol and the other active ingredient i.e., risedronic acid (or its salt) or ibandronic acid (or its salt).

In accordance with an aspect of the present invention, there is provided a pharmaceutical composition for preventing or treating osteoporosis to be administered once a month, the pharmaceutical composition comprising: (a) cholecalciferol-containing granules obtained by adsorbing on microcrystalline cellulose a solution prepared by dissolving (i) cholecalciferol in an amount corresponding to 24,000 to 50,000 IU, preferably 30,000 IU; (ii) one or more selected from tocopheryl acetate, butylhydroxytoluene, and butylhydroxyanisol as a first stabilizing agent; and (iii) a binder in ethanol or an aqueous solution of ethanol, (b) mannitol as a second stabilizing agent, and (c) risedronic acid or its salt or ibandronic acid or its salt.

The component (a), i.e. cholecalciferol-containing granules, are prepared by adsorbing a solution prepared by dissolving cholecalciferol, the first stabilizing agent, and the binder in ethanol or the aqueous solution of ethanol on microcrystalline cellulose. The cholecalciferol-containing granules may be prepared by performing a granulation process comprising: mixing a solution prepared by dissolving cholecalciferol, the first stabilizing agent, and the binder in ethanol or the aqueous solution of ethanol with microcrystalline cellulose in a mixer to prepare a slurry, and then drying and milling the slurry. A ratio of ethanol to water in the aqueous solution of ethanol is not be limited as long as the cholecalciferol, the first stabilizing agent, and the binder are easily dissolved. For example, the aqueous solution of ethanol may be a 95 wt% aqueous solution of ethanol. The ethanol or aqueous solution of ethanol may be used in an amount of 0.006 to 0.02 mL/Tab. When such an amount of the ethanol or ethanol aqueous solution is added, cholecalciferol and the first stabilizer may be easily dissolved to form a solution. Then, the binder is added thereto and dissolved, thereby producing a viscous solution, that is, a sticky liquid. The mixer used herein may be a conventional mixer used in the pharmaceutical industry, such as a high speed mixer. As described above, when the sticky liquid in which cholecalciferol, the first stabilizing agent and the binder are dissolved is fed into the high speed mixer, followed by stirring, a slurry may be obtained. Then, the slurry is dried and milled according to a conventional method, thereby obtaining granules. If necessary, the granules may be sifted using a sieve having an appropriate mesh.

The mesh size may be appropriately determined so as to obtain granules having a particle size that is similar to those of mannitol (second stabilizing agent) and the other active ingredient, i.e., risedronic acid (or its salt) or ibandronic acid (or its salt) which are to be mixed with the granules in a subsequent process. For example, the granules may be sifted by using a 30 or 40 mesh sieve.

The first stabilizing agent may be selected from tocopheryl acetate, butylhydroxytoluene, butylhydroxyanisol, and a mixture thereof. A mixture of tocopheryl acetate and butylhydroxytoluene may be preferably used as the first stabilizing agent. A weight ratio of tocopheryl acetate and the butylhydroxytoluene may be in a range of 1 : 0.2 to 1.0. The first stabilizing agent may be present in an amount of 0.4 to 2.0 parts by weight based on 1 part by weight of cholecalciferol.

The binder allows cholecalciferol to be easily adsorbed on microcrystalline cellulose. The binder may be a conventional binder used in the pharmaceutical industry. Examples of the binder are hydroxypropyl cellulose, povidone, hypromellose, etc. Preferably, the binder may be hydroxypropyl cellulose.

In the pharmaceutical composition of the present invention, the second stabilizing agent, i.e., mannitol, may be present in an amount of 5 to 20 wt% based on the total weight of the pharmaceutical composition.

The pharmaceutical composition of the present invention may further comprise a conventional excipient used in the pharmaceutical industry, in addition to the components (a) to (c). For example, the pharmaceutical composition of the present invention may further comprise one or more disintegrating agent selected from the group consisting of crospovidone, sodium starch glycolate, sodium carboxymethyl cellulose, and alginic acid; and one or more lubricant selected from the group consisting of magnesium stearate, stearic acid, talc, and silicon dioxide.

A dosage form of the pharmaceutical composition according to the present invention may be a tablet form, and if necessary, may further comprise a coating layer to form a coated-tablet form. The coating layer may be, for example, a film coating layer comprising a mixture of hydroxypropyl methylcellulose and polyethylene glycol, preferably having a weight ratio of hydroxypropyl methylcellulose and polyethylene glycol in a range of 5 to 15 : 1, more preferably in a range of about 10 : 1. If necessary, the coating layer may additionally comprise a light-shielding agent such as titanium oxide, or a lubricant such as talc.

The coating layer may be formed by homogeneously dispersing and/or dissolving the components described above in an organic solvent such as methylene chloride, isopropyl alcohol, or the like, or water, and then coating on the tablet according to a conventional coating method.

In accordance with an embodiment, there is provided a pharmaceutical composition in a tablet form consisting of (a) cholecalciferol-containing granules comprising 0.75 mg of cholecalciferol powder (40,000 IU/mg), 0.5 mg of butylhydroxytoluene, 0.9 mg of tocopheryl acetate, 6 mg of hydroxypropyl cellulose, and 40 mg of microcrystalline cellulose, (b) 40.0 mg of mannitol, (c) 150 mg of sodium risedronate or 150 mg of sodium ibandronate, and (d) 4 mg of crospovidone and 4 mg of magnesium stearate.

In accordance with another embodiment, there is provided a pharmaceutical composition in a film-coated tablet form comprising a film coating layer consisting of 5.1 mg of hydroxypropyl methylcellulose, 0.51 mg of polyethylene glycol 6000 (PEG 6000), 1.28 mg of titanium oxide, and 0.11 mg of talc on the tablet.

The present invention will be described in further detail with reference to the following examples and experimental examples. These examples and experimental examples are for illustrative purposes only and are not intended to limit the scope of the present invention.

### Examples 1 to 6 and Comparative Examples 1 to 3. Film-coated tablet containing sodium risedronate and cholecalciferol

Film-coated tablets each containing sodium risedronate and cholecalciferol were prepared according to the components and amounts shown in Tables 1 and 2 below. The amounts shown in Tables 1 and 2 are amounts per one tablet.

Cholecalciferol powder (40,000 IU/mg), a first stabilizing agent (butylhydroxytoluene, butylhydroxyanisol, and/or DL-α-tocopheryl acetate), and hydroxypropyl cellulose were completely dissolved in ethanol under stirring. The resulting solution and microcrystalline cellulose were mixed in a high speed mixer for about 30 seconds to obtain a slurry. The obtained slurry was uniformly spread on a dry plate and then dried at a temperature of about 40., followed by milling with a mill (Fitz mill), thereby obtaining granules. The granules were sifted with a 40 mesh sieve to obtain the granules in which cholecalciferol was adsorbed on the surface of the microcrystalline cellulose. The granules of Comparative Example 2 were prepared without using the first stabilizing agent.

The granules obtained in the above were mixed with sodium risedronate, and mannitol or lactose (Comparative Examples 1 and 2), and then crospovidone and magnesium stearate were added thereto. The resulting mixture was homogeneously mixed, and then compressed to prepare uncoated tablets.

While rotating the uncoated tablets in a film coater at a speed of about 15 rpm, a coating solution obtained by homogeneously dissolving and dispersing a mixture of hydroxypropyl methylcellulose (hypromellose), polyethylene glycol 6000, titanium oxide, and talc in a mixed solvent of isopropyl alcohol and methylene chloride (a weight ratio of 1 : 1.2) was sprayed thereto to form a film coating layer, thereby preparing a film-coated tablet.

The film-coated tablet of Comparative Example 3 was prepared as follows: The oily DL-α-tocopheryl acetate was completely dissolved in ethanol under stirring, and then hydroxypropyl cellulose was added thereto and dissolved. The resulting solution and microcrystalline cellulose were mixed in a high speed mixer for about 30 seconds to obtain a slurry. The obtained slurry was uniformly spread on a dry plate and then dried at a temperature of about 40., followed by milling with a mill (Fitz mill), thereby obtaining granules. The granules were sifted with a 40 mesh sieve to obtain the granules in which DL-α-tocopheryl acetate was adsorbed on the surface of the microcrystalline cellulose. The granules obtained in the above were mixed with cholecalciferol powder (40,000 IU/mg), the first stabilizing agent (butylhydroxytoluene), sodium risedronate, and mannitol, and then crospovidone and magnesium stearate were added thereto. The resulting mixture was homogeneously mixed, and then compressed to prepare uncoated tablets. While rotating the uncoated tablets in a film coater at a speed of about 15 rpm, a coating solution obtained by homogeneously dissolving and dispersing a mixture of hydroxypropyl methylcellulose (hypromellose), polyethylene glycol 6000, titanium oxide, and talc in a mixed solvent of isopropyl alcohol and methylene chloride (a weight ratio of 1 : 1.2) was sprayed thereto to form a film coating layer, thereby preparing a film-coated tablet.

**[Table 1]**

| Components | Example 1 | Example 2 | Example 3 | Example 4 |
|---|---|---|---|---|
| Sodium risedronate | 150 mg | 150 mg | 150 mg | 150 mg |
| Cholecalciferol powder (40,000 IU/mg) | 0.75 mg | 0.75 mg | 0.75 mg | 0.75 mg |
| Butylhydroxytoluene | 0.5 mg | 0.5 mg | 0.5 mg | - |
| Butylhydroxyanisol | - | - | - | 0.5 mg |
| DL-α-tocopheryl acetate | - | - | 0.9 mg | 0.9 mg |
| Hydroxypropyl cellulose | 6.0 mg | 6.0 mg | 6.0 mg | 6.0 mg |
| Microcrystalline cellulose | 40.0 mg | 40.0 mg | 40.0 mg | 40.0 mg |
| D-mannitol | 20.0 mg | 40.0 mg | 40.0 mg | 40.0 mg |
| Lactose | - | - | - | - |
| Crospovidone | 4.0 mg | 4.0 mg | 4.0 mg | 4.0 mg |
| Magnesium stearate | 4.0 mg | 4.0 mg | 4.0 mg | 4.0 mg |
| Hypromellose | 5.10 mg | 5.10 mg | 5.10 mg | 5.10 mg |
| Polyethylene glycol 6000 | 0.51 mg | 0.51 mg | 0.51 mg | 0.51 mg |
| Titanium oxide | 1.28 mg | 1.28 mg | 1.28 mg | 1.28 mg |
| Talc | 0.11 mg | 0.11 mg | 0.11 mg | 0.11 mg |

**[Table 2]**

| Components | Example 5 | Example 6 | Comparative Example 1 | Comparative Example 2 | Comparative Example 3 |
|---|---|---|---|---|---|
| Sodium risedronate | 150 mg | 150 mg | 150 mg | 150 mg | 150 mg |
| Cholecalciferol powder (40,000 IU/mg) | 0.75 mg | 0.75 mg | 0.75 mg | 0.75 mg | 0.75 mg |
| Butylhydroxytoluene | 0.5 mg | 0.5 mg | 0.5 mg | - | 0.5 mg |
| Butylhydroxyanisol | - | - | - | - | - |
| DL-α-tocopheryl acetate | 1.8 mg | 5.0 mg | - | - | 0.9 mg |
| Hydroxypropyl cellulose | 6.0 mg | 6.0 mg | 6.0 mg | - | 6.0 mg |
| Microcrystalline cellulose | 40.0 mg | 40.0 mg | 40.0 mg | 40.0 mg | 40.0 mg |
| D-mannitol | 40.0 mg | 40.0 mg | - | - | 40.0 mg |
| Lactose | - | - | 40.0 mg | 40.0 mg | - |
| Crospovidone | 4.0 mg | 4.0 mg | 4.0 mg | 4.0 mg | 4.0 mg |
| Magnesium stearate | 4.0 mg | 4.0 mg | 4.0 mg | 4.0 mg | 4.0 mg |
| Hypromellose | 5.10 mg | 5.10 mg | 5.10 mg | 5.10 mg | 5.10 mg |
| Polyethylene glycol 6000 | 0.51 mg | 0.51 mg | 0.51 mg | 0.51 mg | 0.51 mg |
| Titanium oxide | 1.28 mg | 1.28 mg | 1.28 mg | 1.28 mg | 1.28 mg |
| Talc | 0.11 mg | 0.11 mg | 0.11 mg | 0.11 mg | 0.11 mg |

### Examples 7 to 9. Film-coated tablet containing sodium ibandronate and cholecalciferol

Film-coated tablets each containing sodium ibandronate and cholecalciferol were prepared according to the components and amounts shown in Table 3 below. The amounts shown in Tables 3 are amounts per one tablet.

Cholecalciferol powder (40,000 IU/mg), a first stabilizing agent (butylhydroxytoluene, butylhydroxyanisol, and/or DL-α-tocopheryl acetate), and hydroxypropyl cellulose were completely dissolved in ethanol under stirring. The resulting solution and microcrystalline cellulose were mixed in a high speed mixer for about 30 seconds to obtain a slurry. The obtained slurry was uniformly spread on a dry plate and then dried at a temperature of about 40., followed by milling with a mill (Fitz mill), thereby obtaining granules. The granules were sifted with a 40 mesh sieve to obtain the granules in which cholecalciferol was adsorbed on the surface of the microcrystalline cellulose.

The granules obtained in the above were mixed with sodium ibandronate, and mannitol, and then crospovidone and magnesium stearate were added thereto. The resulting mixture was homogeneously mixed, and then compressed to prepare uncoated tablets.

While rotating the uncoated tablets in a film coater at a speed of about 15 rpm, a coating solution obtained by homogeneously dissolving and dispersing a mixture of hydroxypropyl methylcellulose (hypromellose), polyethylene glycol 6000, titanium oxide, and talc in a mixed solvent of isopropyl alcohol and methylene chloride (a weight ratio of 1 : 1.2) was sprayed thereto to form a film coating layer, thereby preparing a film-coated tablet.

**[Table 3]**

| Components | Example 7 | Example 8 | Example 9 |
|---|---|---|---|
| Sodium ibandronate | 150 mg | 150 mg | 150 mg |
| Cholecalciferol powder (40,000 IU/mg) | 1.25 mg | 1.25 mg | 1.25 mg |
| Butylhydroxytoluene | 0.5 mg | 0.5 mg | 0.5 mg |
| DL-α-tocopheryl acetate | - | 0.9 mg | 5.0 mg |
| Hydroxypropyl cellulose | 6.0 mg | 6.0 mg | 6.0 mg |
| Microcrystalline cellulose | 40.0 mg | 40.0 mg | 40.0 mg |
| D-mannitol | 40.0 mg | 40.0 mg | 40.0 mg |
| Crospovidone | 4.0 mg | 4.0 mg | 4.0 mg |
| Magnesium stearate | 4.0 mg | 4.0 mg | 4.0 mg |
| Hypromellose | 5.10 mg | 5.10 mg | 5.10 mg |
| Polyethylene glycol 6000 | 0.51 mg | 0.51 mg | 0.51 mg |
| Titanium oxide | 1.28 mg | 1.28 mg | 1.28 mg |
| Talc | 0.11 mg | 0.11 mg | 0.11 mg |

### Experimental Example 1: Stability Test

Stability tests of the film-coated tablets prepared in Examples 1 to 9 and Comparative Examples 1 to 3 were performed. All tablets were exposed to the condition of 50. for 2 weeks, and on the seventh and fourteenth days, amounts of the active ingredients in the tablets, i.e., amounts of sodium risedronate or sodium ibandronate acid and cholecalciferol, were measured.

### (1) Amounts of sodium risedronate or sodium ibandronate

The amounts of sodium risedronate or sodium ibandronate measured on the seventh and fourteenth days according to the stability tests are shown in Table 4.

**Table 4**

| | Initial | After 1 week | After 2 weeks |
|---|---|---|---|
| Example 1 | 100.0% | 100.0% | 100.0% |
| Example 2 | 100.0% | 100.1% | 100.0% |
| Example 3 | 100.0% | 100.0% | 99.8% |
| Example 4 | 100.0% | 99.7% | 99.5% |
| Example 5 | 100.0% | 100.0% | 99.7% |
| Example 6 | 100.0% | 100.0% | 99.9% |
| Example 7 | 100.0% | 100.0% | 99.5% |
| Example 8 | 100.0% | 99.7% | 99.7% |
| Example 9 | 100.0% | 99.8% | 99.9% |
| Comparative Example 1 | 100.0% | 99.8% | 99.8% |
| Comparative Example 2 | 100.0% | 99.8% | 100.0% |
| Comparative Example 3 | 100.0% | 99.9% | 99.7% |

From the results shown in Table 4, it can be confirmed that the stabilities of sodium risedronate and sodium ibandronate were maintained in the formulations of the present invention as well as those of Comparative Examples.

### (2) Amounts of cholecalciferol

The amounts of cholecalciferol measured on the seventh and fourteenth days according to the stability tests are shown in Table 5.

**Table 5**

| | Initial | After 1 week | After 2 weeks |
|---|---|---|---|
| Example 1 | 100.0% | 98.5% | 97.8% |
| Example 2 | 100.0% | 99.2% | 98.9% |
| Example 3 | 100.0% | 99.8% | 99.5% |
| Example 4 | 100.0% | 99.6% | 99.3% |
| Example 5 | 100.0% | 99.9% | 99.8% |
| Example 6 | 100.0% | 100.0% | 99.9% |
| Example 7 | 100.0% | 99.7% | 99.5% |
| Example 8 | 100.0% | 100.0% | 99.7% |
| Example 9 | 100.0% | 100.0% | 99.9% |
| Comparative Example 1 | 100.0% | 92.7% | 86.6% |
| Comparative Example 2 | 100.0% | 45.7% | 5.8% |
| Comparative Example 3 | 100.0% | 95.5% | 90.0% |

From the results of Table 5, it can be confirmed that the formulations according to the present invention retain excellent stability without significant content change in cholecalciferol even after being exposed to the condition of 50. for 2 weeks. Also, the greater amount of mannitol leads to the higher stability (Example 1 vs. Example 2); the use of tocopheryl acetate as a first stabilizing agent was desirable (Example 2 vs. Examples 3-6); and the use of a combination of tocopheryl acetate and butylhydroxytoluene was more desirable (Example 3 vs. Example 4-6). In contrast, if the first stabilizing agent and/or the second stabilizing agent (that is, mannitol) were not included, the stabilities were significantly decreased. In particular, if the first stabilizing agent was not used (Comparative Example 2), the stability was markedly decreased. And also, although having the same components, the formulation obtained by simple mixing cholecalciferol and the first stabilizing agent without dissolving in a solvent (ethanol) shows significantly lower stability (Example 3 vs. Comparative Example 3). Therefore, it is desirable to perform a formulation process using the granules, the granules of which are obtained by dissolving cholecalciferol and the first stabilizing agent at the same time and then adsorbing on microcrystalline cellulose through hydroxypropyl cellulose as a binder. Regarding a complex formulation with sodium ibandronate, it can be seen that the use of a combination of tocopheryl acetate and butylhydroxytoluene as a first stabilizing agent is desirable (Examples 7 to 9).

## Claims

1. A pharmaceutical composition for preventing or treating osteoporosis to be administered once a month, the pharmaceutical composition comprising:
(a) cholecalciferol-containing granules obtained by adsorbing on microcrystalline cellulose a solution prepared by dissolving (i) cholecalciferol in an amount corresponding to 24,000 to 50,000 IU; (ii) one or more selected from tocopheryl acetate, butylhydroxytoluene, and butylhydroxyanisol as a first stabilizing agent; and (iii) a binder in ethanol or an aqueous solution of ethanol,
(b) mannitol as a second stabilizing agent, and
(c) risedronic acid or its salt or ibandronic acid or its salt, wherein the components (a), (b) and (c) are homogenously mixed in the composition.

2. The pharmaceutical composition of claim 1, wherein the cholecalciferol is present in an amount corresponding to 30,000 IU.

3. The pharmaceutical composition of claim 1, wherein the cholecalciferol-containing granules are prepared by performing a granulation process comprising: mixing a solution prepared by dissolving cholecalciferol, the first stabilizing agent, and the binder in ethanol or the aqueous solution of ethanol with microcrystalline cellulose in a mixer to prepare a slurry, and then drying and milling the slurry.

4. The pharmaceutical composition of claim 1, wherein the first stabilizing agent is a mixture of tocopheryl acetate and butylhydroxytoluene.

5. The pharmaceutical composition of claim 4, wherein a weight ratio of tocopheryl acetate and butylhydroxytoluene is in a range of 1 : 0.2 to 1.0.

6. The pharmaceutical composition of claim 1, wherein the first stabilizing agent is present in an amount of 0.4 to 2.0 parts by weight based on 1 part by weight of cholecalciferol.

7. The pharmaceutical composition of claim 1, wherein the binder is hydroxypropyl cellulose.

8. The pharmaceutical composition of any one of claims 1 to 7, wherein the mannitol is present in an amount of 5 to 20 wt% based on the total weight of the pharmaceutical composition.

9. The pharmaceutical composition of any one of claims 1 to 7, further comprising one or more disintegrating agent selected from the group consisting of crospovidone, sodium starch glycolate, sodium carboxymethyl cellulose, and alginic acid; and one or more lubricant selected from the group consisting of magnesium stearate, stearic acid, talc, and silicon dioxide.

10. The pharmaceutical composition of any one of claims 1 to 7, wherein a dosage form of the pharmaceutical composition is a tablet form.

11. The pharmaceutical composition of claim 10, further comprising a film coating layer comprising a mixture of hydroxypropyl methylcellulose and polyethylene glycol.

12. The pharmaceutical composition of claim 11, wherein a weight ratio of hydroxypropyl methylcellulose and polyethylene glycol is in a range of 5 to 15 : 1.

13. The pharmaceutical composition of claim 1, wherein the pharmaceutical composition is in a tablet form consisting of (a) cholecalciferol-containing granules comprising 0.75 mg of cholecalciferol powder (40,000 IU/mg), 0.5 mg of butylhydroxytoluene, 0.9 mg of tocopheryl acetate, 6 mg of hydroxypropyl cellulose, and 40 mg of microcrystalline cellulose, (b) 40.0 mg of mannitol, (c) 150 mg of sodium risedronate or 150 mg of sodium ibandronate, and (d) 4 mg of crospovidone and 4 mg of magnesium stearate.

14. The pharmaceutical composition of claim 13, wherein the pharmaceutical composition is in a film-coated tablet form further comprising a film coating layer consisting of 5.1 mg of hydroxypropyl methylcellulose, 0.51 mg of polyethylene glycol 6000 (PEG 6000), 1.28 mg of titanium oxide, and 0.11 mg of talc on the tablet.

## Patentansprüche

1. Pharmazeutische Zusammensetzung zum Verhindern oder Behandeln von Osteoporose, die einmal pro Monat verabreicht wird, die pharmazeutische Zusammensetzung umfassend:
(a) Cholecalciferol enthaltenes Granulat, das durch Absorbieren einer Lösung auf mikrokristalliner Cellulose erhalten wird, wobei die Lösung durch Auflösen von (i) Cholecalciferol in einer Menge, die 24.000 bis 50.000 IE entspricht; (ii) einem oder mehreren ausgewählt aus Tocopherylacetat, Butylhydroxytoluen und Butylhydroxyanisol als erstes Stabilisierungsmittel; und (iii) einem Bindemittel in Ethanol oder einer wässrigen Lösung von Ethanol hergestellt wird,
(b) Mannitol als zweites Stabilisierungsmittel und
(c) Risedronsäure oder deren Salz bzw. Ibandronsäure oder deren Salz, worin die Komponenten (a), (b) und (c) homogen in der Zusammensetzung eingemischt werden.

2. Pharmazeutische Zusammensetzung nach Anspruch 1, worin das Cholecalciferol in einer Menge vorhanden ist, die 30.000 IE entspricht.

3. Pharmazeutische Zusammensetzung nach Anspruch 1, worin das Cholecalciferol enthaltene Granulat hergestellt wird durch ein Granulationsverfahren, umfassend: Mischen einer Lösung hergestellt durch Auflösen von Cholecalciferol, dem ersten Stabilisierungsmittel, und dem Bindemittel in Ethanol oder der wässrigen Lösung von Ethanol mit mikrokristalliner Cellulose in einem Mischer zur Herstellung einer Aufschlämmung und anschließendem Trocknen und Mahlen der Aufschlämmung.

4. Pharmazeutische Zusammensetzung nach Anspruch 1, worin das erste Stabilisierungsmittel ein Gemisch aus Tocopherylacetat und Butylhydroxytoluen ist.

5. Pharmazeutische Zusammensetzung nach Anspruch 4, worin das Gewichtsverhältnis von Tocopherylacetat und Butylhydroxytoluen in einem Bereich von 1: 0,2 bis 1,0 liegt.

6. Pharmazeutische Zusammensetzung nach Anspruch 1, worin das erste Stabilisierungsmittel in einer Menge von 0,4 bis 2,0 Gewichtsteilen bezogen auf 1 Gewichtsteil Cholecalciferol vorhanden ist.

7. Pharmazeutische Zusammensetzung nach Anspruch 1, worin das Bindemittel Hydroxylpropylcellulose ist.

8. Pharmazeutische Zusammensetzung nach einem der Ansprüche 1 bis 7, worin das Mannitol in einer Menge von 5 bis 20 Gew.-% des Gesamtgewichts der pharmazeutischen Zusammensetzung vorhanden ist.

9. Pharmazeutische Zusammensetzung nach einem der Ansprüche 1 bis 7, die ferner ein oder mehrere Desintegrationsmittel ausgewählt aus der Gruppe bestehend aus Crospovidon, Natriumstärkeglycolat, Natriumcarboxymethylcellulose und Algensäure; und ein oder mehrere Schmiermittel ausgewählt aus der Gruppe bestehend aus Magnesiumstearat, Stearinsäue, Talk und Siliciumdioxid umfasst.

10. Pharmazeutische Zusammensetzung nach einem der Ansprüche 1 bis 7, worin eine Darreichungsform der pharmazeutischen Zusammensetzung eine Tablette ist.

11. Pharmazeutische Zusammensetzung nach Anspruch 10, ferner umfassend eine Filmbeschichtungsschicht, die ein Gemisch aus Hydroxypropylmethylcellulose und Polyethylenglycol umfasst.

12. Pharmazeutische Zusammensetzung nach Anspruch 11, worin ein Gewichtsverhältnis von Hydroxypropylmethylcellulose und Polyethylenglycol in einem Bereich von 5 bis 15: 1 liegt.

13. Pharmazeutische Zusammensetzung nach Anspruch 1, worin die pharmazeutische Zusammensetzung in einer Tablettenform vorliegt bestehend aus (a) Cholecalciferol enthaltend Granulat, das 0,75 mg Cholecalciferol-Pulver (40.000 IE/mg), 0,5 mg Butylhydroxytoluen, 0,9 mg Tocopherylacetat, 6 mg Hydroxypropylcellulose und 40 mg mikrokristalliner Cellulose umfasst, (b) 40,0 mg Mannitol, (c) 150 mg Natriumrisedronat oder 150 mg Natriumibandronat und (d) 4 mg Crospovidon und 4 mg Magnesiumstearat.

14. Pharmazeutische Zusammensetzung nach Anspruch 13, worin die pharmazeutische Zusammensetzung in Form einer filmbeschichteten Tablette vorliegt, ferner umfassend eine Filmbeschichtungsschicht bestehend aus 5,1 mg Hydroxypropylmethylcellulose, 0,51 mg Polyethylenglycol 6000 (PEG 6000), 1,28 mg Titanoxid und 0,11 mg Talk auf der Tablette.

## Revendications

1. Composition pharmaceutique pour la prévention ou le traitement de l'ostéoporose pour être administrée une fois par mois, la composition pharmaceutique comprenant :
(a) des granules contenant du cholécalciférol obtenues par adsorption sur de la cellulose microcristalline, une solution préparée par la dissolution (i) du cholécalciférol en une quantité correspondante à 24 000 à 50 000 UI ; (ii) un ou plusieurs choisis parmi de l'acétate de tocophéryle, du butylhydroxytoluène, et du butylhydroxyanisole comme un premier agent stabilisant ; et (iii) un liant dans de l'éthanol ou une solution aqueuse d'éthanol,
(b) du mannitol comme un deuxième agent stabilisant, et
(c) de l'acide risédronique ou son sel ou de l'acide ibandronique ou son sel, où les composants (a), (b) et (c) sont mélangés de manière homogène dans la composition.

2. Composition pharmaceutique selon la revendication 1, dans laquelle le cholécalciférol est présent en une quantité correspondante à 30 000 UI.

3. Composition pharmaceutique selon la revendication 1, dans laquelle les granules contenant du cholécalciférol sont préparées en réalisant un procédé de granulation comprenant : le mélange d'une solution préparée par la dissolution du cholécalciférol, du premier agent stabilisant, et du liant dans de l'éthanol ou dans la solution aqueuse d'éthanol avec de la cellulose microcristalline dans un mixeur pour préparer une boue, et ensuite le séchage et le broyage de la boue.

4. Composition pharmaceutique selon la revendication 1, dans laquelle le premier agent stabilisant est un mélange d'acétate de tocophéryle et de butylhydroxytoluène.

5. Composition pharmaceutique selon la revendication 4, dans laquelle un rapport massique d'acétate de tocophéryle et de butylhydroxytoluène se trouve dans une gamme de 1 : 0,2 à 1,0.

6. Composition pharmaceutique selon la revendication 1, dans laquelle le premier agent stabilisant est présent en une quantité de 0,4 à 2, 0 parts en poids sur base de 1 part en poids de cholécalciférol.

7. Composition pharmaceutique selon la revendication 1, dans laquelle le liant est de l'hydroxypropylcellulose.

8. Composition pharmaceutique selon l'une quelconque des revendications 1 à 7, dans laquelle le mannitol est présent en une quantité de 5 à 20 % en poids sur base du poids total de la composition pharmaceutique.

9. Composition pharmaceutique selon l'une quelconque des revendications 1 à 7, comprenant en outre un ou plusieurs agent désintégrant choisi parmi le groupe constitué de crospovidone, de glycolate d'amidon sodique, de carboxyméthylcellulose sodique, et d'acide alginique ; et un ou plusieurs lubrifiants choisis parmi le groupe constitué de stéarate de magnésium, d'acide stéarique, de talc, et de dioxyde de silicium.

10. Composition pharmaceutique selon l'une quelconque des revendications 1 à 7, dans laquelle une forme de dosage de la composition pharmaceutique est sous forme de comprimés.

11. Composition pharmaceutique selon la revendication 10, comprenant en outre une couche de revêtement à film comprenant un mélange d'hydroxypropylméthylcellulose et de polyéthylène glycol.

12. Composition pharmaceutique selon la revendication 11, dans laquelle un rapport massique d'hydroxypropylméthylcellulose et de polyéthylène glycol se trouve dans une gamme de 5 à 15: 1.

13. Composition pharmaceutique selon la revendication 1, dans laquelle la composition pharmaceutique est sous forme de comprimés constitués de (a) granules contenant du cholécalciférol comprenant 0,75 mg de poudre de cholécalciférol (40 000 UI/mg), 0,5 mg de butylhydroxytoluène, 0,9 mg d'acétate de tocophéryle, 6 mg d'hydroxypropylcellulose, et 40 mg de cellulose microcristalline, (b) 40,0 mg de mannitol, (c) 150 mg de risédronate sodique ou 150 mg d'ibandronate sodique, et (d) 4 mg de crospovidone et 4 mg de stéarate de magnésium.

14. Composition pharmaceutique selon la revendication 13, dans laquelle la composition pharmaceutique est sous forme de comprimé pelliculé comprenant en outre une couche de revêtement à film constitué de 5,1 mg d'hydroxypropylméthylcellulose, 0,51 mg de polyéthylène glycol 6000 (PEG 6000), 1,28 mg d'oxyde de titane, et 0,11 mg de talc sur le comprimé.
